# EUROPEAN PATENT APPLICATION

(11) **EP 3 278 791 A1**
(43) Date of publication of application: **07.02.2018**
(21) Application number: 16773120.7
(22) Date of filing: 31.03.2016
(51) Int. Cl.: A61K 9/00, A61K 47/34, A61L 31/00, D01D 5/08, D01F 1/10, D01F 6/92, D04H 1/728, D04H 3/011

(54) **METHOD FOR MANUFACTURING DRUG-CONTAINING BIODEGRADABLE FIBER MATERIAL BY ELECTROSPINNING**

(30) Priority: 31.03.2015 US 201562140722 P
(71) Applicant: Orthorebirth Co., Ltd., Yokohama-shi, Kanagawa 224-0033 (JP)
(72) Inventor: NISHIKAWA, Yasutoshi, Yokohama-shi Kanagawa 224-0033 (JP); MAKITA, Masashi, Yokohama-shi Kanagawa 224-0033 (JP); OSADA, Naoki, Yokohama-shi Kanagawa 224-0033 (JP)
(74) Representative: J A Kemp
(86) International application number: PCT/JP2016/060670
(87) International publication number: WO 2016/159240

(57) **Abstract**

The present invention addresses the problem of providing a drug formulation material with which localized sustained release of a drug at any site in the body is possible, and which has good bioabsorption and is absorbed and broken down by the body after sustained release of the drug. A drug formulation material that has an exceedingly high sustained release effect, and that solves the foregoing problem, was successfully developed by dissolving a biodegradable resin and a drug in a solvent to prepare a spinning solution, and spinning fibers from the spinning solution by electrospinning.

## Description

### TECHNICAL FIELD

This application claims priority to U.S. Provisional Application No. 62/140722 filed on Mar. 31, 2015, the entirety of which is herein incorporated by reference.

The present invention relates to a method for preparing a thermoplastic biodegradable resin (e.g., polylactic acid or PLGA) containing a powdery drug (e.g., inorganic particles for bone formation, an anticancer agent, or an antibiotic) as a spinning solution for electrospinning.

The present invention further relates to a method for manufacturing biodegradable fibers by electrospinning using a spinning solution for electrospinning prepared by the above method.

The present invention further relates to a method for efficiently collecting biodegradable fibers prepared by the above method as non-woven fabric or cotton.

The present invention relates to an in-vivo locally implantable sustained-release agent including a bioabsorbable cotton-like material manufactured by the above manufacturing method; and usage thereof (treatment method).

The drug-containing bioabsorbable cotton-like material according to the present invention has excellent local sustained releasability and is quickly absorbed and broken down by the body after the release of a medicinal ingredient, and is therefore extremely effective as a drug formulation other than an orally administered agent, such as a long-acting injection (depot preparation) or an in-vivo implantable drug formulation.

### BACKGROUND ART

Among drug administration routes, oral administration accounts for about 60%, which is the most widely used administration route. However, peptide/protein drugs have recently increased, which are polymeric drugs that cannot be expected to have adequate absorbability and stability by oral administration. Further, in the future, gene/nucleic acid drugs having higher molecular weights are expected to be clinically used. Further, when orally administered, a drug is absorbed from the small intestine and flows throughout the body via the bloodstream. For this reason, oral administration is not suitable for drugs required to have locality and sustained releasability.

As administration routes other than oral administration, there are various administration routes such as nasal administration, pulmonary administration, ocular instillation, rectal administration, transdermal administration, and administration by injection. Injections are used next to orally administered agents. However, among injections, intravenous drip injections cannot often be expected to have the effect of localized sustained release as in the case of oral administration. Further, injections are quickly absorbed but often have a problem with the persistence of medicinal effect after administration.

On the other hand, long-acting injections (depot preparations) have also been developed. Long-acting injections are injections produced so that their medicinal effects last for several days to several months per administration. Such long-acting injections are often used as hormonal drugs, and applied in the form of oil-based injections or suspended injection. They are applied also to antipsychotic agents that are likely to have a problem with continuous oral administration. They are percutaneously or intramuscularly injected and are expected to have a sustained effect even after administration, but it is difficult to allow a drug to locally act only on a target site (tissue).

As a drug formulation that sustains the effect of a physiological active substance for a long time, microspheres using a biodegradable polymer (multinuclear microcapsules) have also been researched (JP-A-6-211648). Microspheres usually refer to a spherical preparation having a particle diameter of about several micrometers, and those having a particle diameter of 1 µm or less are sometimes called nanospheres. Microspheres satisfy the localized sustained releasability of a drug and locality, but are suspended in a liquid on the condition that they are administered by injection (e.g. subcutaneous injection). Therefore, microspheres have a problem with drug stability (the drug is dispersed), and therefore their research and development are underway even now.

In order to prevent the dispersion of a drug and maintain stability, a drug formulation using a hydrophobic polymer (silicone that is a non-biodegradable polymer) as a carrier has also been studied (JP-A-2001-199879). In this case, the dispersion of a drug can be prevented, but there is a problem that the carrier is left in the body. There is no problem in applying the drug formulation to a site from which the drug formulation is relatively easily removed, such as a site under the skin, but implantation of the drug formulation in any site in the body has a risk.

On the other hand, in the field of bone regeneration materials, a method has been performed in which a material prepared by adding an osteogenic factor to fibers made of a biodegradable resin such as polylactic acid is implanted into a bone defect (U.S. Patent Publication No. 2011-000952). The biodegradable fibers are hydrolyzed by contact with body fluid after implanted in the body so that a drug contained in the biodegradable fibers is sustainably released and the biodegradable fibers are absorbed by the body with the lapse of time and disappear. Therefore, bone formation is effectively achieved while a burden on a patient is reduced.

Electrospinning is used as a method for manufacturing fibers from a biodegradable resin in the above-described method. In the electrospinning method, a spinning solution is extruded from a nozzle as fibers by an electrostatic attractive force generated in an electric field. Therefore, such a spinnable solution needs to be prepared.

In the field of bone regeneration materials, spinning solutions for electrospinning have heretofore been prepared by dissolving biodegradable resins using solvents.

### CITATION LIST

### PATENT LITERATURE

PATENT LITERATURE 1: JP-A-6-211648
PATENT LITERATURE 2: JP-A-2001-199879
PATENT LITERATURE 3: U.S. Patent No. 6689374
PATENT LITERATURE 4: U.S. Patent Publication No. 2011-0009522
PATENT LITERATURE 5: WO 2015/005205

### NON-PATENT LITERATURES

NON-PATENT LITERATURE 1: Walsh et al. B-TCP bone graft substitutes in a bilateral rabbit tibial defect model. Biomaterials 29 (2008) 266-271
NON-PATENT LITERATURE 2: Obata et al. Electrospun microfiber meshes of silicon-doped vaterite/poly(lactic acid) hybrid for guided bone regeneration. Acta Biometatialla 6 (2010) 1248-1257
NON-PATENT LITERATURE 3: Fujiwara et al. Guided bone regeneration membrane made of polycaprolactone/calcium carbonate composite nano-fibers. Biomaterials 26 (2005) 4139-4147)
NON-PATENT LITERATURE 4:Hench LL. Polak JM: Third-generation biomedical materials. Science 2002, 295: 1014-1017)
NON-PATENT LITERATURE 5: Weiss et al. Targeted expression of MYCN causes neuroblastoma in transgenic mice. The EMBO Journal Vol. 16 No. 11 pp. 2985-2995, 1997
NON-PATENT LITERATURE 6: Kishida et al. Midkine Promotes Neuroblastoma through Notch 2 Signaling. Cancer Res; 73 (4) 1318-1327, 2013

### SUMMARY OF THE INVENTION

### TECHNICAL PROBLEM

It is therefore an object of the present invention to provide a drug formulation material that is capable of locally and sustainably releasing a drug at any site in the body, that has bioabsorbability, and that is absorbed and broken down by the body after sustained release of the drug.

### SOLUTION TO PROBLEM

As a method for locally administering a drug to an affected area, a method has been proposed (U.S. Patent No. 6689374) in which a material obtained by adding a drug to biodegradable fibers is implanted in an affected area to sustainably release the drug. A spinning solution that can be used for electrospinning is prepared by first dispersing fine particles in water or a solvent and then uniformly dispersing the dispersion liquid in a solution prepared by dissolving a biodegradable resin to mix them. However, when a large amount of fine powder particles are contained, these fine particles cannot be easily uniformly dispersed in the spinning solution by such a method. Therefore, the biodegradable fibers have a small outer diameter and a short sustained release period.

The present inventors have intensively studied, and as a result have found that the above problem can be solved by 1) dissolving a biodegradable resin and a drug in a solvent to prepare a spinning solution and 2) spinning fibers from the spinning solution by electrospinning. Based on the finding, the present inventors have successfully developed a drug formulation material that includes a fibrous material having a large diameter and has a very high sustained release effect.

More specifically, the present invention includes the following aspects [1] to [20].
[1] A bioabsorbable cotton-like material having a cotton- or nonwoven fabric-like structure, including a fibrous material that includes a drug and a biodegradable resin and has an average outer diameter of 1 µm or more but 150 µm or less, preferably 10 µm or more but 150 µm or less, more preferably 30 µm or more but 110 µm or less, even more preferably 60 µm or more but 110 µm or less.
[2] The bioabsorbable cotton-like material according to [1], wherein the fibrous material has an average molecular weight of 50000 or more but less than 1000000, preferably 50000 or more but less than 500000, more preferably 60000 or more but less than 400000.
[3] The bioabsorbable cotton-like material according to [1], whose bulk density when dried or hydrated is 0.01g/cm³ to 0.1 g/cm³, more preferably 0.01 g/cm³ to 0.05 g/cm³.
[4] The bioabsorbable cotton-like material according to any one of [1] to [3], wherein the biodegradable resin is PLGA or a copolymer thereof.
[5] The bioabsorbable cotton-like material according to any one of [1] to [4], wherein the drug is an anticancer agent.
[6] The bioabsorbable cotton-like material according to any one of [1] to [5], which has been subjected to sterilization treatment.
[7] A method for manufacturing a bioabsorbable cotton-like material, characterized by including:
   step 1) dissolving a biodegradable resin and a drug in a solvent to prepare a spinning solution; and
   step 2) spinning fibers from the spinning solution by electrospinning.
[8] The method for manufacturing a bioabsorbable cotton-like material according to [7], wherein in the step 2), fibers are spun by electrospinning by applying a voltage between a nozzle part provided on a solution extrusion side and a plate placed in an ethanol bath provided on a collector side to deposit a bioabsorbable cotton-like material in the ethanol bath to form a bioabsorbable cotton-like material having a cotton-like three-dimensional structure.
[9] The method for manufacturing a bioabsorbable cotton-like material according to [7] or [8], further including step 3) of sterilization treatment.
[10] The method for manufacturing a bioabsorbable cotton-like material according to any one of [7] to [9], wherein the biodegradable resin is PLGA or a copolymer thereof, and the solvent is chloroform or dichloromethane.
[11] The method for manufacturing a bioabsorbable cotton-like material according to any one of [7] to [10], wherein the drug is an anticancer agent.
[12] A method of treating or preventing a disease in the patient, comprising:
   1) implanting the bioabsorbable cotton-like material according to [6] in a body of patient;
   2) sustainably releasing the drug from the bioabsorbable cotton-like material; and
   3) treating or preventing the disease in the patient by an effect of the sustainably released drug.
[13] The method according to [10], wherein the bioabsorbable cotton-like material according to [6] is implanted by laparotomy.
[14] The method according to [12], wherein the bioabsorbable cotton-like material according to [6] is implanted by a minimally invasive medical procedure using an injector.
[15] The method according to [12], wherein the drug is an anticancer agent, and the disease is cancer.
[16] The method according to [15], wherein the patient has been subjected to resection of cancer tissue or cancer cells.
[17] The method according to [15] or [16], wherein the cancer is malignant bone tumor.
[18] A kit for use in the method according to [12] or [13], including the bioabsorbable cotton-like material according to [6].
[19] A kit for use in the method according to [14], including an injector and the bioabsorbable cotton-like material according to [6].
[20] The kit according to [19], wherein the bioabsorbable cotton-like material according to [6] is contained in the injector.

The "biodegradable resin (biodegradable polymer)" is generally defined as a "resin that can be used in the same manner as common plastics under normal use conditions but is broken down after use and finally converted into carbon dioxide and water and returned to nature". In the present invention, the biodegradable resin means a resin (polymer) broken down by the bodies of humans and non-human mammals (including domestic animals such as cattle and pigs and companion animals such as dogs and cats). The biodegradable resin is not particularly limited, but may be a natural polymer such as cellulose or starch or any one of several types of biodegradable synthetic polymers having excellent biocompatibility and adjusted biodegradation rate and mechanical strength. Examples of the synthetic polymers include: polyglycolic acid (PGA) and polylactic acid (PLA) (poly-L-lactic acid: PLLA, poly-D-lactic acid: PDLA); copolymers thereof; [polylactic acid-polyglycolic acid copolymer (poly(lactide-co-glycolide) copolymer) (PLGA)]; and polydioxanone (PDS). In the case of PLGA, the ratio of monomers PLA and PGA can be changed depending on a desired degradation rate. The ratio may be PLA: PGA= 90:10, 85:15, 80:20, 75:25, 70:30, 65:35, 60:40, 55:45, 50:50, 45:55, 40:60, 35:65, 30:70, 25:75, 20:80, 15:85, or 10:90.

The "solvent" is not particularly limited as long as it is a volatile solvent that has low solubility in water and is a good solvent for polymers. Examples of such a solvent include chloroform, methylene chloride, and carbon tetrachloride. Alternatively, a mixed solvent of such a solvent and a solvent compatible therewith (e.g., ethyl ether or ethyl acetate) may be used. The solvent is preferably one that does not impair the activity of the "drug".

The "drug" means an inorganic or organic material that can be administered to a human body by adding to biodegradable fibers and that exerts its activity when the biodegradable fibers are implanted in a human body.

Examples of the drug include, but are not limited to, an anticancer agent, an antibiotic, a polypeptide having a physiological activity (e.g., influenza vaccine or insulin), an antipyretic agent, an analgesic, an immunostimulating agent, an immunosuppressive agent, an antiinflammatory agent, a cough suppressant, an antiepileptic agent, an antihistamine, an antihypertensive diuretic, an antidiabetic agent, a muscle relaxant, an antiulcer agent, an antidepressant, an antiallergic agent, an antianginal agent, an arrhythmia therapeutic agent, a vasodilator, an anticoagulant, a hemostatic agent, an antitubercular agent, a narcotic antagonist, and a hormonal agent. The "drug" may include not only drugs in the pharmaceutical field but also drugs in the cosmetics field (e.g., vitamins, placenta, and hyaluronic acid). The drug is preferably resistant to the "solvent" described above.

The "bulk density" is measured with reference to JIS L 1097 of cotton.

The "electrospinning (ES)" refers to a method for manufacturing microfibers, in which a high voltage is applied between a polymer solution contained in a syringe and a collector electrode so that the solution extruded from the syringe is electrically charged and adhered to the collector as microfibers.

The "minimally invasive medical procedure" refers to a procedure in which, in surgery, not only the size of a surgical cut on the body but also a physical and mental burden on a patient is smaller as compared to a conventional procedure (e.g., laparotomy). Endoscopic surgery corresponds to the minimally invasive medical technique.

The "injector" (inserter) refers to a device that is percutaneously inserted into the body under X-ray fluoroscopy or the like to leave a drug or medical instrument contained therein in the body. An endoscope or the like may be connected to the injector.

Examples of the method of "sterilization treatment" include radiation sterilization (gamma rays, electron beams), ethylene oxide gas sterilization, and high-pressure steam sterilization. In the present invention, radiation sterilization with γ rays is preferably used. When radiation sterilization with γ rays of 25 kGy to 35 kGy is performed, the average molecular weight decreases (60000 to 100000).

### ADVANTAGEOUS EFFECTS OF INVENTION

The present invention is effective as a method for manufacturing biodegradable fibers carrying a drug from a biodegradable resin containing the drug by electrospinning.

The biodegradable fibers can provide a drug formulation material that is capable of locally and sustainably releasing a drug at any site in the body, has bioabsorbability, and is absorbed and broken down by the body after sustained release of the drug.

Further, implantation of the drug formulation in a patient can produce a therapeutic/preventive effect to enhance QOL (Quality of Life).

### BRIEF DESCRIPTION OF DRAWINGS

FIG. 1A is a SEM photograph of fibers (40TCP-30SiV-30PLLA) spun by a method described in Reference Example 1.
FIG. 1 (B) shows fibers spun from a solution having the same mixing ratio as in Reference Example 1 but not subjected to kneading with a kneader.
FIG. 2 is a SEM photograph of fibers (70TCP-30PLLA) spun by a method described in Reference Example 2.
FIG. 3 shows fibers of PLLA 100% spun using an electrospinning device in Reference Example 3.
FIG. 4 shows the configuration of an electrospinning device used in the present invention.
FIG. 5 is a SEM photograph of fibers (30-fold amount of carboplatin-PLGA) spun in Example 1.
FIG. 6 Methods for calculating a compression rate and a recovery rate
FIG. 7 Results of Example 2
FIG. 8 Shapes
FIG. 9 Results of Example 3
FIG. 10 Results of Example 4: Sustained-release behavior of carboplatin from a cotton-like carrier Carboplatin was sustainably released over 168 hours.
FIG. 11 Results of Example 5: Images of a dissected mouse that developed cancer and died at 8 weeks of age.
   There was a very large tumor enough to fill the gap between the left and right kidneys.
FIG. 12 Results of Example 5: Images of a dissected mouse (F166) implanted with the cotton-like carrier and euthanized at 12 weeks of age No tumor was observed. The cotton-like carrier remained.
FIG. 13 Results of Example 5: Changes in body weights of mice after implantation The body weights of mice implanted with the cotton-like carrier increased similarly to those of sham surgery mice (implanted with a cotton-like material made of only a polymer and carrying no anticancer agent), suggesting that side effects of an anticancer agent did not occur.
FIG. 14 Results of Example 5: Abdominal ganglia was excised and fixed with formalin to be histologically evaluated.
FIG. 15 Results of Example 5: H&E stained sections On the left side of the aorta (indicated by an yellow arrow), scarring of fibroblasts (having an elongated nuclear shape) is observed which is not usually observed.
FIG. 16 Results of Example 5: H&E stained sections Calcified portions (circular portions that look like missing portions: indicated by blue arrows) are observed.
FIG. 17 Results of Example 5: A mouse to which the same amount of carboplatin as contained in the cotton-like carrier was directly intraperitoneally administered The mouse moved slowly and trembled, and its lower abdomen was obviously thin. The intestines were necrosed and became dysfunctional. Ingested diet was collected in the stomach, but the small and large intestines contained nothing.
FIG. 18 Results of Example 5: A mouse to which PBS was directly intraperitoneally administered. The behavior and appearance of the mouse were both normal. There was no abnormality in the internal organs.

### DESCRIPTION OF EMBODIMENTS

In one embodiment of the present invention, a small amount of a drug is added to and mixed with a solution obtained by dissolving a biodegradable resin in a solvent to prepare a spinning solution, and fibers are spun by electrospinning using the spinning solution. Polylactic acid or PLGA can be dissolved in a solvent to prepare a spinning solution for electrospinning. Therefore, a spinning solution prepared by mixing a very small amount of a drug into a solution of polylactic acid or PLGA can be spun into fibers by electrospinning.

In one embodiment of the present invention, a syringe of an electrospinning device is filled with the solution obtained above as a spinning solution to extrude the solution from a nozzle as yarns. The yarns extruded from the nozzle fly in a parabola toward a grounded electrode as a target and are deposited on a collector. The collector is formed in a net shape and contained in a container filled with an ethanol solution. The yarns extruded from the nozzle enter the surface of the ethanol solution and precipitate in the solution at their entry points. The precipitated yarns are deposited on the collector and form a nonwoven fabric- or cotton-like material. The "cotton-like material" refers to a material that can be deformed by hands (shape processability); that can be torn into pieces and again put the pieces together after tear (size processability); that can be restored after compression (elastic force); and that can be squeezed by hands to adjust its hydration amount.

In one embodiment of the present invention, the bottom surface (about 15 cm x 25 cm) of a collector of an electrospinning device is used as an electrode plate when fibers are extruded from a nozzle. The collector does not use an ethanol solution. This method makes it possible to deposit fibers on the collector in the form of non-woven fabric.

### EXAMPLES

The present invention may be described in more detail with reference to the following examples, comparative examples, and reference examples, but is not limited to these examples.

### <Reference Example 1> PLLA, β-tricalcium phosphate, and Si-containing vaterite phase calcium carbonate (40TCP-30SiV-30PLLA)

### Step 1

Drugs and polylactic acid are mixed and kneaded by a kneader. The kneader is preheated to a set temperature of 170 to 190°C, and then 15 g of poly-L-lactic acid pellets (PURAC PL24, molecular weight: 200000 to 300000, melting point: 175 to 185°C) are fed into the kneader and heated and kneaded at a set temperature of 180°C to 190°C for about 4 minutes. Then, a powder obtained by mixing 20 g of β-tricalcium phosphate powder and 15 g of SiV powder is fed into the kneader, and the mixture is further kneaded at the same set temperature for about 10 minutes.

When heated at a set temperature of 180°C to 190°C of the kneader, the mixture can be kneaded by applying torque by the kneader in that state. Although the state of poly-L-lactic acid heated by the kneader is not exactly clear, the present inventors estimate that there are a melted part that has reached the melting point of poly-L-lactic acid and a part in a softened state on the verge of melting.

In the present invention, even when poly-L-lactic acid is heated but stays in a softened state without reaching a melted state, fine powder particles can be uniformly dispersed in the matrix resin as long as the matrix resin can be kneaded in a softened state by applying torque by the kneader.

The powder of β-tricalcium phosphate and the powder of SiV added later are mixed with poly-L-lactic acid by kneading so that the fine particles are uniformly dispersed in the poly-L-lactic acid resin. Although the dispersion state at the molecular level is not exactly clear, it can be considered, based on the findings of the present inventors and the like, that β-tricalcium phosphate and SiV are immobilized in polylactic acid as a matrix resin by the coordinate bond between the calcium ion of β-tricalcium phosphate and the carboxyl group of poly-L-lactic acid and the amido bond between an amino group included in SiV and the carboxyl group.

### Step 2

A composite of the drugs and polylactic acid is prepared. Then, the obtained kneaded product of β-tricalcium phosphate, SiV, and poly-L-lactic acid is taken out of the kneader and allowed to stand at ordinary temperature for cooling. In this way, a composite lump of poly-L-lactic acid and the drugs is obtained.

### Step 3

The composite lump obtained above is dissolved in a solvent (e.g., chloroform) to prepare a spinning solution whose poly-L-lactic acid concentration is about 10%. The dissolution of the composite lump in a solvent is performed by placing the composite lump in a container filled with chloroform and slowly rotating the composite lump using a magnetic stirrer for about 5 hours for stirring.

### Step 4

A syringe (diameter: 15.8 mm, extrusion speed: 15 mL/h) of an electrospinning device (e.g., NANON manufactured by Mecc) is filled with the spinning solution prepared above, and fibers are extruded through a nozzle (syringe needle: 18G) by applying a voltage of about 30 kV, and are deposited on a collector while the nozzle is moved a width of 200 mm at a speed of 40 mm/sec and the needle tip is cleaned at an interval of 2 minutes (*) (condition in chamber = temperature: 30 centigrade temperature or less; humidity: 50% or less; length from needle tip to device floor: 37 cm).

(*) The interval between automatic cleaning to remove the drop of solution formed at the tip of the needle.

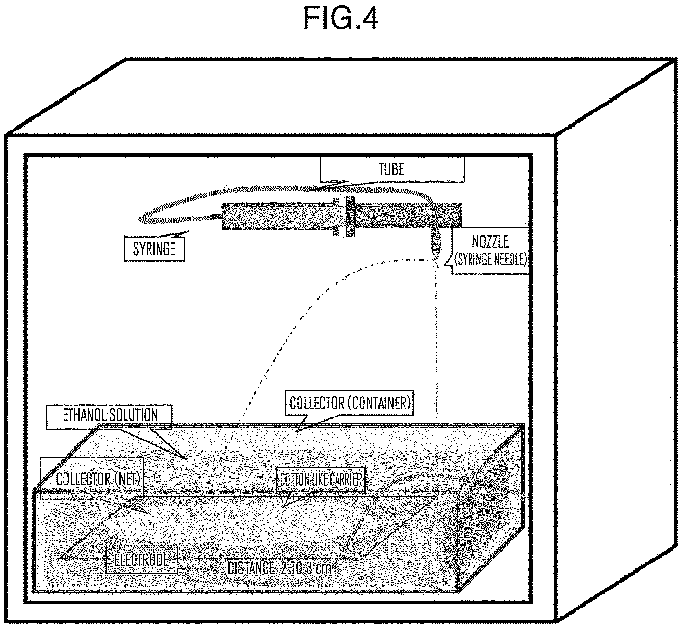

As shown in FIG. 4, in electrospinning, an electrode is provided on the collector side to guide yarns extruded from the nozzle toward the electrode. The collector is contained in a container filled with an ethanol solution, and the yarns guided from the nozzle toward the electrode fly in a parabola, enter the surface of the ethanol solution, and precipitate in the ethanol solution at their entry points. The precipitated yarns are deposited on the mesh of the collector formed in a net-like shape and form a cotton-like material. The yarns extruded from the nozzle during spinning are widely deposited on the surface of the collector by reciprocally moving the nozzle a constant distance at a constant speed on a rail, which is effective at increasing a collection rate.

### Results

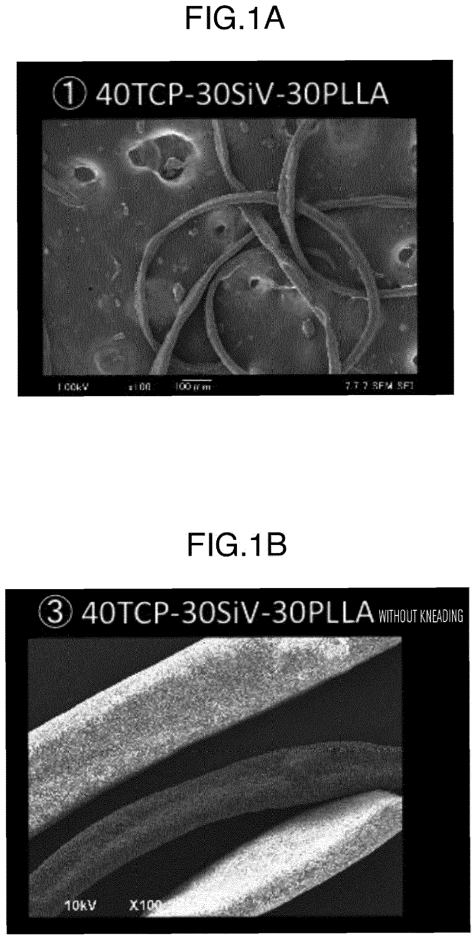

The average diameter of the fibers extruded from the nozzle by electrospinning was about 50 µm. The SEM photograph of the obtained fibers is shown in FIG. 1 (A). For comparative reference, FIG. 1 (B) shows the result of an attempt to spin fibers by the electrospinning device from a solution prepared to have the same composition as in Reference Example 1 without the process of kneading by a kneader. At least fibrous material was obtained, but had a much larger diameter than fibers manufactured by electrospinning.

### <Reference Example 2> β-tricalcium phosphate and PLLA (70TCP-30PLLA)

### Step 1

β-tricalcium phosphate and poly-L-lactic acid (PURAC PL24, molecular weight: 200000 to 300000) are kneaded by a kneader.

The kneader is preheated to a set temperature of 170 to 190°C for 3 minutes, and then 15 g of poly-L-lactic acid pellets are fed into the kneader and heated and kneaded at a set temperature of 180°C to 190°C for about 4 minutes. Then, 35 g of β-tricalcium phosphate powder is fed into the kneader, and the mixture is further kneaded at the same set temperature for about 10 minutes.

When heated at a set temperature of 180°C to 190°C of the kneader, the mixture can be kneaded by applying torque by the kneader in that state. The state of poly-L-lactic acid heated by the kneader is not exactly clear. The present inventors estimate that there are a melted part that has reached the melding point of poly-L-lactic acid and a part in a softened state on the verge of melting.

The powder of β-tricalcium phosphate added later is mixed well with poly-L-lactic acid by kneading, and is therefore uniformly dispersed in the poly-L-lactic acid resin. As for the dispersion state, the present inventors estimate that β-tricalcium phosphate is immobilized in polylactic acid as a matrix resin by the coordinate bond between the calcium ion of β-tricalcium phosphate and the carboxyl group of poly-L-lactic acid.

### Step 2

A composite of β-tricalcium phosphate and polylactic acid is prepared.

Then, the obtained kneaded product of β-tricalcium phosphate and poly-L-lactic acid is taken out of the kneader and allowed to stand at ordinary temperature for cooling. In this way, a composite lump of poly-L-lactic acid and TCP is obtained.

### Step 3

The composite lump of PLLA and β-tricalcium phosphate obtained above is dissolved in a solvent (e.g., chloroform) to prepare a spinning solution whose PLLA concentration is about 10 wt%. The dissolution of the composite lump in a solvent is performed by placing the composite lump in a container filled with a solvent (e.g., chloroform) and slowly rotating the composite lump using a magnetic stirrer for about 5 hours for stirring.

### Step 4

A syringe of an electrospinning device is filled with the spinning solution. The spinning solution is extruded from a nozzle as fibers, and the fibers are deposited on a collector.

As shown in FIG. 4, in electrospinning, an electrode is provided on the collector side to guide yarns extruded from the nozzle toward the electrode. The collector is contained in a container filled with an ethanol solution, and the yarns guided from the nozzle toward the electrode fly in a parabola, enter the surface of the ethanol solution, and precipitate in the ethanol solution at their entry points. The precipitated yarns are deposited on the mesh of the collector formed in a net-like shape and form a cotton-like material.

### Results

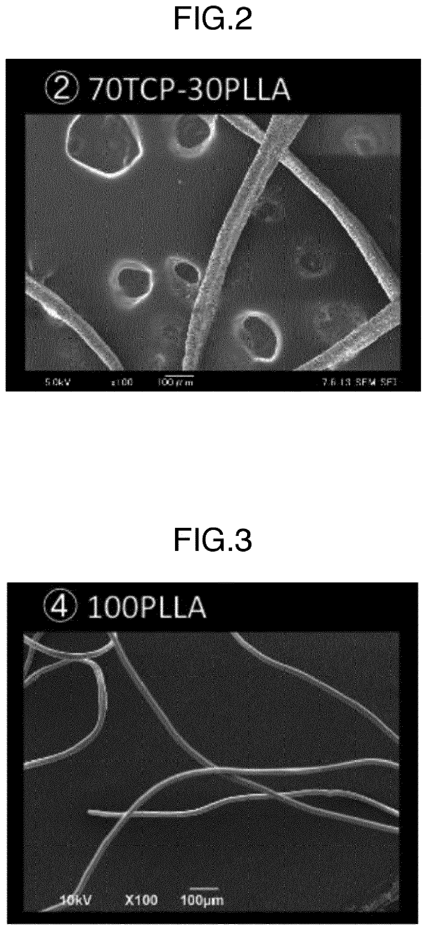

The diameters of the fibers extruded from the nozzle by electrospinning were not stable as compared to the case of PLLA-βTCP-SiV described above, and were about 65 to 80 µm.
The SEM photograph of the obtained fibers is shown in FIG. 2.

### <Reference Example 3> PLLA 100%

FIG. 3 shows fibers spun by electrospinning from a biodegradable resin composed of 100% PLLA used in Reference Examples 1 and 2 under the same conditions.

It is expected that when the amount of a drug to be mixed is very small, similar fibers can be spun by electrospinning.

### <Reference Example 4> PLGA and SiV (50SiV-50PLGA)

### Step 1

A drug and PLGA are kneaded by a kneader.

The kneader is heated to a set temperature of 160 to 165°C for 3 minutes, and then 25 g of PLGA pellets (molar ratio: 82:18, melting point: 130 to 140°C) are fed into the kneader and heated and kneaded at a set temperature of 160°C to 165°C for about 4 minutes. Then, 25 g of SiV powder is fed into the kneader, and the mixture is further kneaded at the same set temperature for about 10 minutes.

When heated at a set temperature of 160°C to 165°C of the kneader, the mixture can be kneaded by applying torque by the kneader in that state. The state of PLGA heated by the kneader is not exactly clear. The present inventors estimate that there are a melted part that has reached the melding point of poly-L-lactic acid and a part in a softened state on the verge of melting.

Even when poly-L-lactic acid is heated but stays in a softened state without reaching a melted state, fine powder particles can be uniformly dispersed in the matrix resin as long as the matrix resin can be kneaded in a softened state by applying torque by the kneader.

The powder of SiV added later is mixed well with PLGA by kneading, and is therefore uniformly dispersed in the matrix resin. As for the dispersion state, the present inventors estimate that SiV is immobilized in polylactic acid as a matrix resin by the coordinate bond between the carboxyl group of PLGA and the calcium of calcium carbonate and the amido bond between the carboxyl group of PLGA and an amino group included in SiV.

### Step 2

A composite of SiV and PLGA is prepared.

Then, the obtained kneaded product of SiV and PLGA is taken out of the kneader and allowed to stand at ordinary temperature for cooling. In this way, a composite lump of PLGA and a drug is obtained.

### Step 3

The composite lump of PLGA and SiV obtained above is dissolved in a solvent (e.g., chloroform) to prepare a spinning solution whose PLGA concentration is about 13 to 15 wt%. The dissolution of the composite lump in a solvent is performed by placing the composite lump in a container filled with a solvent (e.g., chloroform) and slowly rotating the composite lump using a magnetic stirrer for about 5 hours for stirring.

### Step 4

A syringe of an electrospinning device is filled with the spinning solution. The spinning solution is extruded from a nozzle as fibers, and the fibers are deposited on a collector.

In electrospinning, an electrode is provided on the collector side to guide yarns extruded from the nozzle toward the electrode. The collector is contained in a container filled with an ethanol solution, and the yarns guided from the nozzle toward the electrode fly in a parabola, enter the surface of the ethanol solution, and precipitate in the ethanol solution at their entry points. The precipitated yarns are deposited on the mesh of the collector formed in a net-like shape and form a cotton-like material.

### <Comparative Example 1> Production Results of TCP-SiV-PLLA

The present inventors tried to produce fibers under the same conditions as in Reference Example 1 except that the ratio among PLLA, β-tricalcium phosphate, and Si-containing vaterite phase calcium carbonate was changed. The following Table 1 shows conditions under which fibers were successfully produced, and the following Table 2 shows conditions under which fibers were unsuccessfully produced.

**[TABLE 1]**

| Kneader | | | | | ES | | |
|---|---|---|---|---|---|---|---|
| Mixing ratio wt% | | | Temperature | Result | Concentration | Voltage | Result |
| PLLA | TCP | SiV | | | | | |
| 70 | 0 | 30 | 180°C | ○ | 8% | 20kV | ○ |
| 50 | 0 | 50 | 180°C | ○ | 8% | 20kV | ○ |
| 30 | 60 | 10 | 180°C | ○ | 10% | 29kV | ○ |
| 30 | 40 | 30 | 180°C | ○ | 10, 8% | 25kV | ○ |
| 30 | 0 | 70 | 180°C | ○ | 8% | 20kV | ○ |

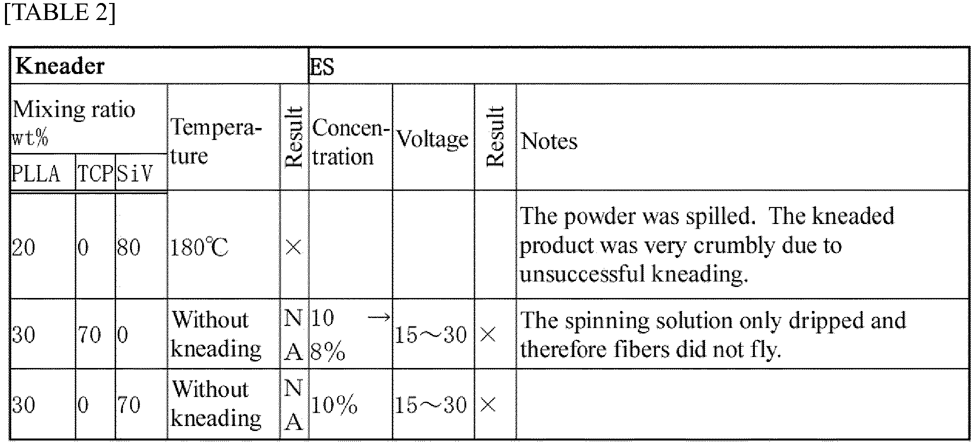

When the proportion of the polymer was 20 wt%, the proportion of the powder was too large to perform kneading. Further, when PLLA was directly dissolved in chloroform without the process of kneading, and a mixture of the solution and TCP or SiV was kneaded, the solution only dripped from the tip of the needle, and therefore spinning could not be performed.

### <Comparative Example 2> Production Results of TCP-SiV-PLGA

The present inventors tried to produce fibers using PLGA (LG855S (manufactured by Evonik, PLLA: PGA = 85:15)) instead of PLLA. The following Table 3 shows conditions under which fibers were successfully produced, and the following Table 4 shows conditions under which fibers were unsuccessfully produced.

**[TABLE 3]**

| Kneader | | | | | ES | | |
|---|---|---|---|---|---|---|---|
| Mixing ratio wt% | | | Temperature | Result | Concen-tration | Voltage | Result |
| PLGA | TCP | SiV | | | | | |
| 50 | 0 | 50 | 165°C | ○ | 10, 8% | 25kV | ○ |
| 50 | 50 | 0 | 165°C | ○ | 8% | 28kV | ○ |
| 50 | 20 | 30 | 165°C | ○ | 8% | 28kV | ○ |
| 30 | 40 | 30 | 165°C | ○ | 8% | 28kV | ○ |
| 30 | 70 | 0 | 165°C | ○ | 8% | 25kV | ○ |
| | | | 165°C | ○ | 8% | 28kV | ○ |
| | | | | | 7% | 25kV | ○ |
| | | | | | 6% | 25kV | Δ |
| 30 | 0 | 70 | 165°C | ○ | 8% | 28kV | ○ |

| | | | | | | | |
|---|---|---|---|---|---|---|---|
| Δ : Slightly crumbly | | | | | | | |

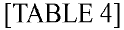

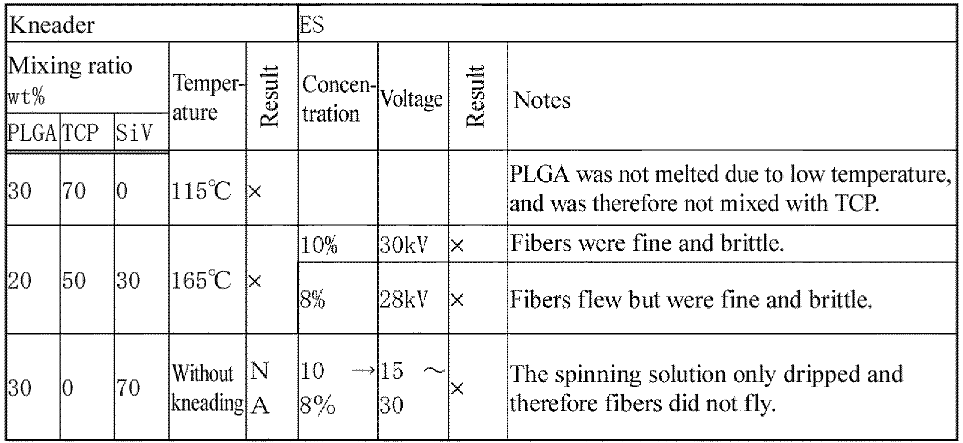

When the proportion of the polymer was 20 wt%, the proportion of the powders was too large to perform kneading. Further, when the kneading temperature was low, the polymer could not be melted and kneaded, and when the process of kneading was omitted, fibers could not be spun.

### <Example 1> PLLA or PLGA, anticancer agent (carboplatin powder, etoposide powder, doxorubicin hydrochloride powder), and antibiotic

Small amounts of an anticancer agent (carboplatin powder, etoposide powder, doxorubicin hydrochloride powder) and an antibiotic were mixed into a solution obtained by dissolving PLLA or PLGA in a solvent to prepare a spinning solution, and fibers were spun from the spinning solution by electrospinning.

### Materials

Biodegradable resin: PLGA (LG855S (manufactured by Evonik, PLLA:PGA= 85:15))
Carboplatin (cis-Diamine(1,1-cyclobutanedicarboxylato)platinum (II)) (CAS number: 41575-94-4, product code: C2043, Tokyo Chemical Industry Co., Ltd.)

### Method

### Step 1

First, 3g of PLGA and carboplatin were dissolved in chloroform to prepare a spinning solution having a PLGA concentration of about 6 wt%. The amount of carboplatin was shown in the following Table 5. A syringe (diameter: 15.8 mm, extrusion speed: 15 mL/h) of an electrospinning device (e.g., NANON, MECC CO., LTD.) was filled with the prepared spinning solution. The spinning solution was extruded as fibers from a nozzle (syringe needle: 18G) by applying a voltage of about 28 kV, and the fibers were deposited on a collector while the nozzle was moved a width of 100 mm to 150 mm at a speed of 40 mm/sec and the needle tip was cleaned at an interval of 2 minutes (condition in chamber = temperature: 30 centigrade temperature or less; humidity: 50% or less; length from needle tip to device floor: 37 cm). The deposited fibers were dried at room temperature to obtain a carboplatin-containing cotton-like material.

**[TABLE 5]**

| Sample name | Amount of polymer | Amount of carboplatin | Concentration of carboplatin | Formation of cotton-like shape |
|---|---|---|---|---|
| 1-fold amount | 3.0 g | 15 mg | 0.50% | Success |
| 10-fold amount | 3.0 g | 150 mg | 4.8% | Success |
| 30-fold amount | 3.0 g | 450 mg | 13% | Success |
| 60-fold amount | 3.0 g | 900 mg | 23% | Failure |

### Results

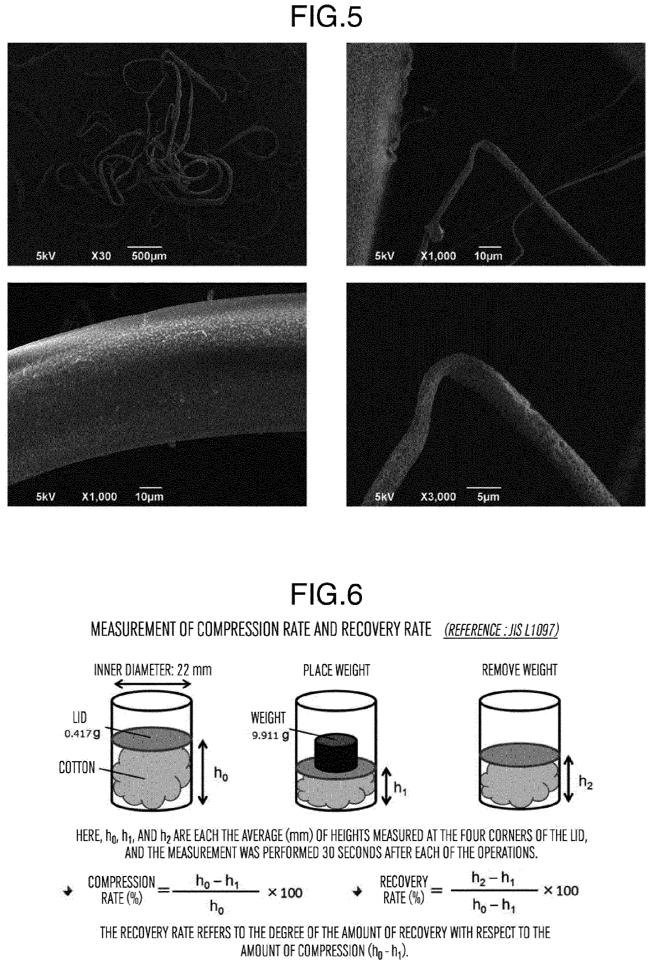

FIG. 5 is a SEM photograph of the obtained carboplatin-containing polylactic acid-glycolic acid copolymer (30-fold amount). The fibers are three-dimensionally intertwined to form a cotton-like material. The fibers are not adhered to each other in the longitudinal direction and form a fluffy three-dimensional cotton-like structure. The fibers had an average outer diameter of 50 µm to 110 µm, and partially had an outer diameter of 1 to 10 µm.

### <Example 2> Measurement of elastic force

The elastic forces of the 30-fold amount of carboplatin-containing polylactic acid-glycolic acid copolymer (hereinafter, referred to as a sample for DDS) prepared in Example 1 and ReBOSSIS (registered trademark) (40TCP-30SiV-30PLLA prepared in Reference Example 1) were measured and compared with the those of ReFit (HOYA Technosurgical Co., Ltd.) and OSferion (Olympus Terumo Biomaterials Corp.) that are approved artificial bone products.

### Materials

The outline of each of the samples used is shown in Table 6.

### [TABLE 6]

**TABLE 6 Outline of Samples of Elastic Force Test**

| Sample name | Components | Shape | Size | Porosity [%] | Hydration amount |
|---|---|---|---|---|---|
| ReBOSSIS | Polylactic acid, *β*-tricalcium phosphate, silicon-containing vaterite | Fibrous | 0.1 g (2.5 ml) | About 98% | 0.8 cc |
| Sample for DDS | Polylactic acid-glycolic acid copolymer, carboplatin | Fibrous | 0.1 g | N/A | 1.6 cc |
| ReFit | Low-crystalline calcium phosphate collagen | Block | 10 × 10 × 10 mm (1.0 ml) | 95% | 1 cc |
| OSferion | *β*-tricalcium phosphate | Block | 10 × 10 × 10 mm (1.0 ml) | 73~82 | 1 cc |

### Method

First, 0.1g of ReBOSSIS or the sample for DDS or 10 × 10 × 10 mm (1.0 mL) of ReFit or OSferion was placed in a transparent tube having an inner diameter of 22 mm. In an experiment under hydration conditions, 0.8 cc, 1.6 cc, and 1 cc of pure water was added to ReBOSSIS, the sample for DDS, and ReFit and OSferion, respectively. A designated lid (0.417 g) was placed on each of the samples. The bulk height at this time was defined as h₀.

Then, a designated weight (9.911 g) was placed on the lid, and the bulk height at this time was defined as h₁.

Finally, the weight was removed, and the bulk height at this time was defined as h₂. The h₀, h₁, or h₂ was determined by calculating the average of heights measured at the four corners of the lid.

Fig. 6 shows the calculation method of a compression rate and the calculation method of a recovery rate.

### Results

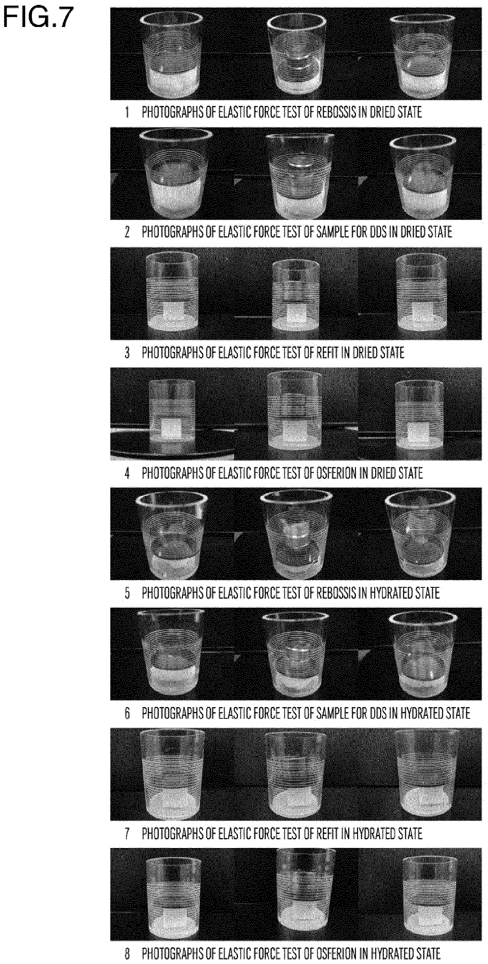

The results of the elastic force test are shown in Table 7 on the next page. The compression rates and recovery rates determined by calculation are shown in Table 8. Further, the photographs of the experiment (left: before adding weight, center: during adding weight, right: after removal of weight) are also shown in FIG. 7.

### [TABLE 7]

**TABLE 7 Results of Elastic Force Test**

| | Dry [mm] | | Hydration [mm] | |
|---|---|---|---|---|
| ReBOSSIS | h₀ | 10.3 | h₀ | 9.2 |
| | h₁ | 6.1 | h₁ | 5.5 |
| | h₂ | 7.3 | h₂ | 5.6 |
| Sample for DDS | h₀ | 14.9 | h₀ | 9.9 |
| | h₁ | 9.1 | h₁ | 8.1 |
| | h₂ | 11.6 | h₂ | 8.5 |
| ReFit | h₀ | 12.0 | h₀ | 12.0 |
| | h₁ | 12.0 | h₁ | 12.0 |
| | h₂ | 12.0 | h₂ | 12.0 |
| OSferion | h₀ | 12.0 | h₀ | 13.0 |
| | h₁ | 12.0 | h₁ | 13.0 |
| | h₂ | 12.0 | h₂ | 13.0 |

### [TABLE 8]

**TABLE 8 Results of Determination of Compression Rate and Recovery Rate**

| **Experimental item** | **Description of experiment** | **State** | **Sample** | **Compression rate(%)** | **Recovery rate(%)** |
|---|---|---|---|---|---|
| Elastic force | Measurement of recovery volume after compression | Dried state | ReBOSSIS | 41 | 27 |
| | | | Sample for DDS | 39 | 44 |
| | | | ReFit | 0 | 0 |
| | | | OSferion | 0 | 0 |
| | | Hydrated state | ReBOSSIS | 39 | 3 |
| | | | Sample for DDS | 14 | 24 |
| | | | ReFit | 0 | 0 |
| | | | OSferion | 0 | 0 |

The compression rate and recovery rate of ReFit or OSferion were both 0, whereas ReBOSSIS or the sample for DDS had certain compression rate and recovery rate.

It is to be noted that when the bulk density of the sample for DDS obtained in Example was measured with reference to JIS L1097, the bulk density in a dried state was 0.0177 g/cm³, and the bulk density in a hydrated state was 0.0266 g/cm³.

The results suggest that the bioabsorbable cotton-like material according to the present invention can be compressed when inserted into an injector or the like, introduced into the body through the injector by a minimally invasive medical procedure, and then quickly recover its volume in the body.

### <Example 3> Shape processability and size processability

In terms of shape processability and size processability, the 30-fold amount of carboplatin-containing polylactic acid-glycolic acid copolymer (hereinafter, referred to as a sample for DDS) prepared in Example 1 and ReBOSSIS (registered trademark) (40TCP-30SiV-30PLLA prepared in Reference Example 1) were compared with ReFit (HOYA Technosurgical) and OSferion (Olympus Terumo Biomaterials Corp.) that are approved artificial bone products.

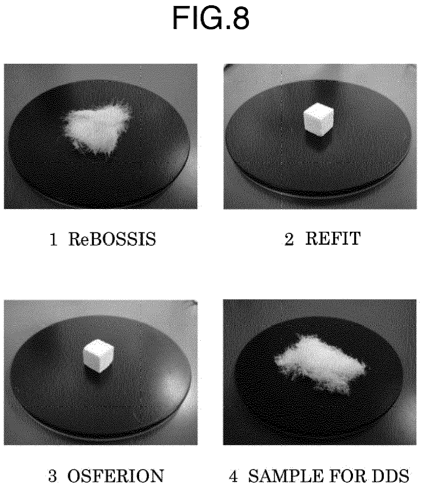

The outline of each of the samples used is shown in Table 9, and the shape of each of the samples is shown in FIG. 8.

### [TABLE 9]

**TABLE 9 Outline of Samples**

| Sample name | Components | Shape | Size | Porosity [%] | Hydration amount |
|---|---|---|---|---|---|
| ReBOSSIS | Polylactic acid, *β*-tricalcium phosphate, silicon-containing vaterite | Cotton-like shape | 1.0 g (25 ml) | About 98% | 8 cc |
| ReFit | Low-crystalline calcium phosphate + collagen | Block | 10 × 10 × 10 mm (1.0 ml) | 95% | 1 cc |
| OSferion | *β*-tricalcium phosphate | Block | 10 × 10 × 10 mm (1.0 ml) | 73∼82 | 1 cc |
| Sample for DDS | PLGA, carboplatin | Block | 1.0 g | NA | 16 cc |

### 1. Shape processability

Whether or not each of the samples could be processed using tools into a shape that could be contained in a cylindrical plastic container having a diameter of 8.5 to 9 mm was determined. The tools used were tweezers, a cutter, and osteotomy scissors. Whether or not each of the samples could be processed using these tools was determined, and the time required to process each of the samples into a cylindrical shape was determined. The shape processability of each of the samples was determined in a dried state and a hydrated state. Please see the above for the samples used and hydration amounts.

### 2. Size processability

Whether or not each of the samples could be torn in half by hands and could be again put the halves together after tear was determined. The shape processability of each of the samples was determined in a dried state and a hydrated state. Please see the above for the samples used and hydration amounts.

### Results

### 1. Shape processability

### [TABLE 10]

**TABLE 10 Results of Examination for Shape Processability**

| | State | Sample | Results | | | Necessary time (s) |
|---|---|---|---|---|---|---|
| | | | Tweezers (manual) | Cutter | Ostectomy scissors | |
| Shape processability | Dried state | ReBOSSIS | ○ | ○ | ○ | 11 |
| | | ReFit | × | ○ | ○ | 181 |
| | | OSferion | × | ○ | ○ | 534 |
| | | Sample for DDS | ○ | ○ | ○ | 10 |
| | Hydrated state | ReBOSSIS | ○ | ○ | ○ | 10 |
| | | ReFit | Δ | ○ | ○ | 96 |
| | | OSferion | × | ○ | ○ | 297 |
| | | Sample for DDS | ○ | ○ | ○ | 14 |

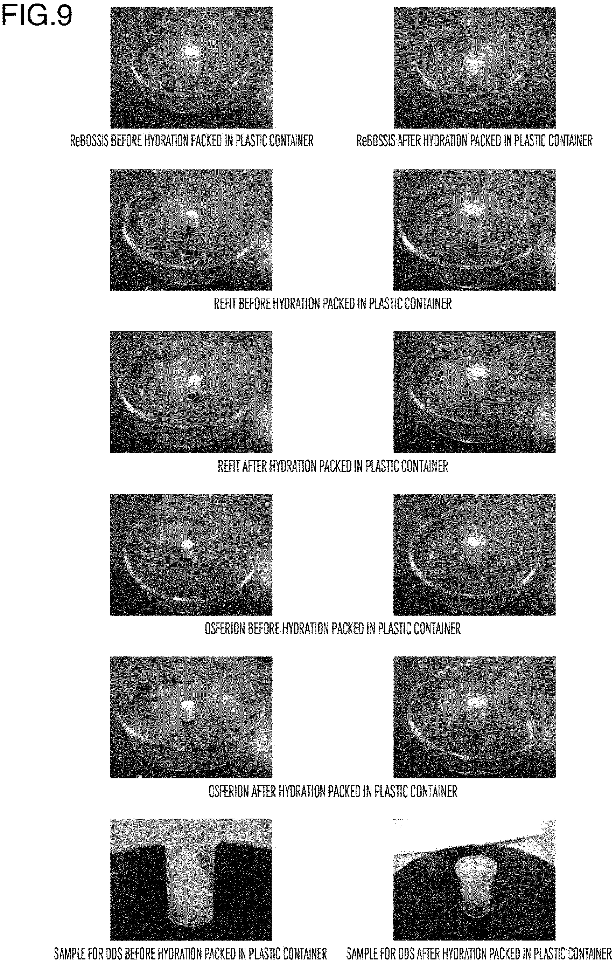

Fig. 9 shows the samples after processing and the samples contained in plastic containers.

ReBOSSIS in a dried state and ReBOSSIS in a hydrated state could be both manually shaped, and could be packed in a plastic container in a short time because their shapes could be easily processed. ReFit in a dried state needed to be processed with a cutter, and therefore it took time for shaping. ReFit in a hydrated state could be relatively quickly shaped because its shape could be changed by hands to some extent. The properties of OSferion were hardly changed even in a hydrated state, and therefore it took time for shaping both in a dried state and a hydrated state.

As in the case of ReBOSSIS, the shape of the sample for DDS could be processed in a short time.

### 2. Size processability

### [TABLE 11]

**TABLE 11 Results of Examination for Size Processability**

| | Item | State | Sample | Result |
|---|---|---|---|---|
| | | Dried state | ReBOSSIS | ○ |
| | | | ReFit | × |
| | | | OSferion | × |
| | | | Sample for DDS | ○ |
| | Whether or not the sample can be torn into pieces | Hydrated state | ReBOSSIS | ○ |
| | | | ReFit | Δ |
| | | | OSferion | × |
| Size processability | | | Sample for DDS | ○ |
| | | Dried state | ReBOSSIS | ○ |
| | | | ReFit | × |
| | | | OSferion | × |
| | Whether or not the pieces can be put together after tear | | Sample for DDS | ○ |
| | | Hydrated state | ReBOSSIS | ○ |
| | | | ReFit | × |
| | | | OSferion | × |
| | | | Sample for DDS | ○ |

ReBOSSIS in a dried state and ReBOSSIS in a hydrated state both could be torn into pieces by hands, and then the pieces could be again put together. ReFit could be torn into pieces by hands after hydration, but could not be torn in an arbitrary shape. Therefore, the degree of freedom of size processing was lower than that of ReBOSSIS. As in the case of ReBOSSIS, the sample for DDS could be torn into pieces by hands, and then the pieces could be again put together.

This result suggests that the bioabsorbable cotton-like material according to the present invention can be very easily shaped so as to fit in the site of implantation.

### <Example 4> Sustained releasability of bioabsorbable cotton-like material carrying anticancer agent

### Method

First, 25 mg of a 30-fold amount of carboplatin-carrying cotton-like material was weighed and placed in each 1.5 cm³ Eppendorf tube. Then, 0.5 cm³ of pure water was added thereto to immerse the cotton-like material in the pure water. At specified time intervals, the cotton-like material was removed with tweezers and transferred into an empty 1.5 cm³ Eppendorf tube. Then, 0.5 cm³ of pure water was newly added to the empty Eppendorf tube to exchange the solution. (The solution was exchanged once in the morning after a lapse of 1 day or more).

The amount of carboplatin at each sampling time was measured with an ultraviolet spectrophotometer. Specifically, 10 µL of the stored solution obtained at each sampling time was weighed and diluted with ultrapure water in a 100 µL cell (10-fold dilution) to measure the amount of carboplatin. In this experiment, the number of samples to be measured was 3 (n = 3). Conditions for measurement of sustained releasability and UV measurement are as follows.

### Measurement conditions

Sampling time: 5 min, 1, 2, 4, 6 h, 1, 2, 3, 4, 7 day
Detection condition: UV (220 nm)

### Results

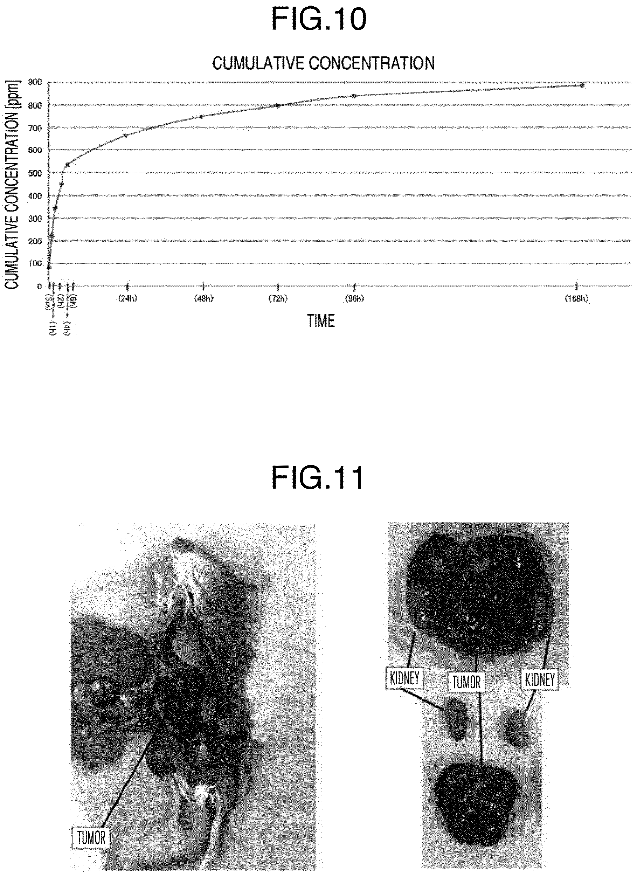

The sustained-release behavior of carboplatin from the cotton-like carrier was observed over 168 hours (FIG. 10).

Therefore, it can be said that the cotton-like carrier is a very excellent drug carrier capable of locally administering an anticancer agent for a long term.

### <Example 5> Effectiveness of bioabsorbable cotton-like material carrying anticancer agent as novel drug delivery (DDS) material

### Method

A homozygous (Tg/Tg) mouse strain was used which was established by backcross of transgenic (Tg) mice that express a MYCN gene from the promoter of Tyrosine Hydroxylase (TH) that is a sympathetic nerve-specific enzyme (NON-PATENT LITERATURE 5: Weiss et al) with 129tTer/SvJcl wild-type mice (CLEA Japan) (NON-PATENT LITERATURE 6: Kishida et al). These mice, in which neural crest cells are fated to differentiate into sympathetic neurons and MYCN is expressed at the timing of expressing TH that is one of markers, spontaneously develop neuroblastoma from the superior mesenteric ganglion that is one of sympathetic ganglia and die at about 7 to 8 to 9 weeks of age. Heterozygous mice develop tumors and die 2 months after birth or later (at 9 to 20 weeks of age) when they reach sexual maturity.

An experiment was performed in which the 30-fold amount of carboplatin-containing polylactic acid-glycolic acid copolymer (bioabsorbable cotton-like material (cotton-like carrier)) prepared in Example 1 was implanted in the abdominal cavity of a homozygous (Tg/Tg) mouse (in the vicinity of the abdominal superior mesenteric ganglion that is a main site where neuroblastoma occurs (between both the kidneys)) according to the following experimental protocol (Table 12), carboplatin was directly administered into the abdominal cavity of a mouse in the same amount as contained in the cotton-like carrier, and phosphate buffered saline (PBS) was administered into the abdominal cavity of a mouse as a control for comparison.

**[TABLE 12]**

| | Male 30_1* | Female 30_1 | Female 30_2 |
|---|---|---|---|
| Mouse No | M169 | F166 | F179 |
| Carrier | 30-fold amount carrier | 30-fold amount carrier | 30-fold amount carrier |
| Amount of carrier implanted | 0. 05g | 0.05g | 0.025g |
| Birth day | 150607 | 150519 | 150716 |
| Start date of implantation | 150708 | 150617 | 150822 |
| End date of implantation | 150831 | 150727 | 151016 |
| Amount of days of living after implantation | 53 | 51 (Euthanasia) | 56 (Euthanasia) |

### Results

The mice that were not implanted with the cotton-like carrier died at 7 to 8 weeks of age, but the mice implanted with the cotton-like carrier continued to live beyond the age of 8 weeks, and F166 and F179 were euthanized at 12 weeks of age.

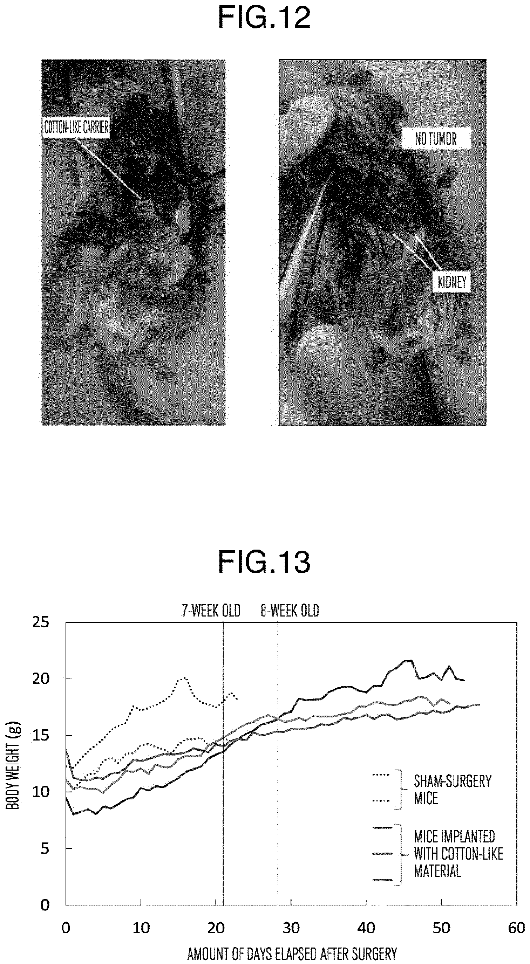

FIG. 11 shows the mouse during dissection which developed cancer and died at 8 weeks of age, and FIG. 12 shows the mouse (F166) during dissection which was implanted with the cotton-like carrier and euthanized at 12 weeks of age.

The changes in the body weights of the mice after implantation surgery are shown in FIG. 13.

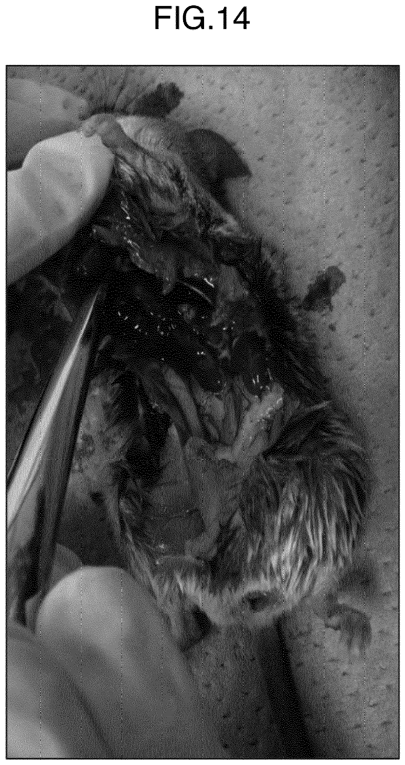

In the mouse shown in FIG. 11, a very large tumor enough to fill the gap between the left and right kidneys was observed, and the mouse obviously died from cancer. On the other hand, in the mouse shown in FIG. 12, a tumor was not observed at all even after a lapse of 12 weeks. Therefore, the abdominal ganglion (F 166) was excised and fixed with formalin (FIG. 14).

As shown in FIG. 12, the cotton-like carrier remained. This is because only 8 weeks (12 week-old) had passed after implantation. It is expected that the cotton-like carrier is entirely absorbed by the body in about a half year after implantation.

As can be seen from FIG. 13, the body weights of the mice implanted with the cotton-like carrier increased similarly to sham-surgery mice. This reveals that the side effects of the anticancer agent did not occur. Even after the sham-surgery mice died from cancer at 8 weeks of age, the body weights of the mice implanted with the cotton-like carrier continued to steadily increase. This suggests that the mice were cured of cancer.

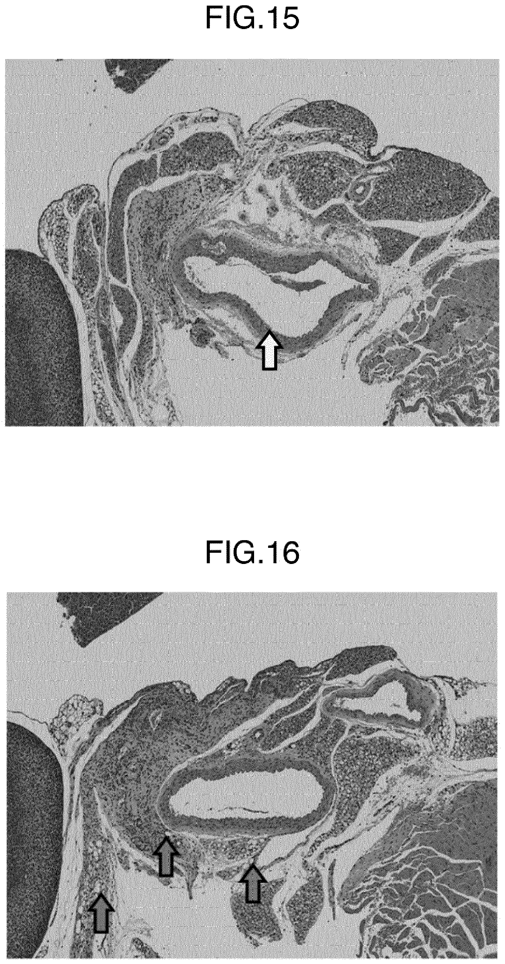

FIGs. 15 and 16 show the H&E-stained sections of the mouse implanted with the cotton-like carrier (FIG. 14).

From FIGs. 15 and 16, the following findings were obtained.
- "Neuroblastoma cells" that had small cell bodies and were poor in cytoplasm were not observed.
- Calcification and scarring with fibroblasts were observed.

From the above, it is estimated that cancer cells were killed by the anticancer effect of the cotton-like material, and the killed cancer cells remained as scarring.

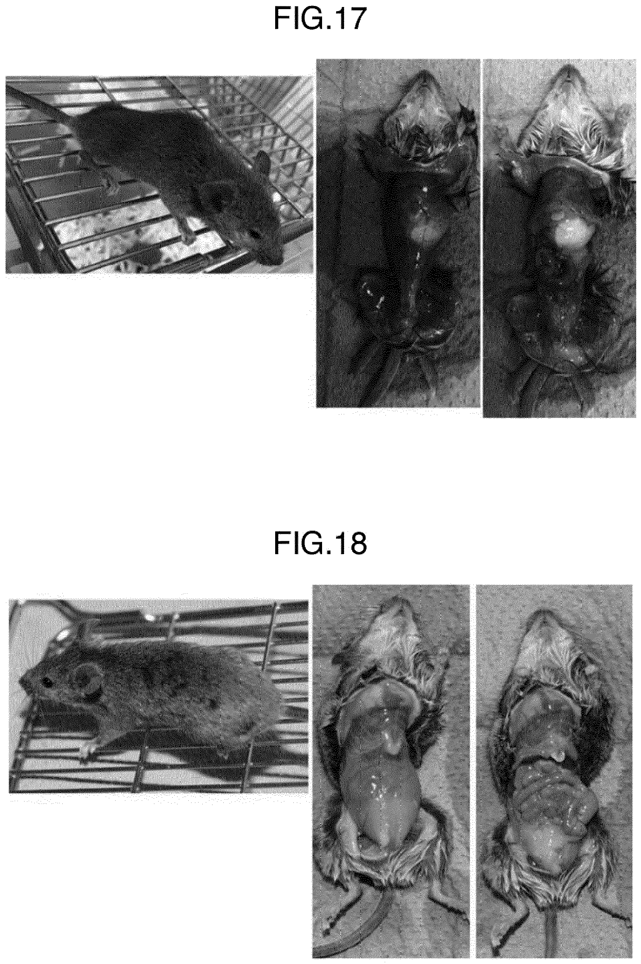

FIG. 17 shows the appearance of the mouse to which carboplatin was directly intraperitoneally administered in the same amount as contained in the cotton-like carrier and the mouse during dissection, and FIG. 18 shows the appearance of the mouse to which PBS was directly administered as a control for comparison and the mouse during dissection.

It is to be noted that the mice to which carboplatin was directly intraperitoneally administered were healthy mice, but all the mice died within several days. On the other hand, the mice to which PBS was administered lived three weeks or more after administration, and were therefore euthanized in the fourth week after administration.

As can be seen from FIG. 17 and FIG. 18, when carboplatin was directly administered in the same amount as contained in the cotton-like carrier, the mice died due to the occurrence of serious side effects.

The LD50 (50% lethal dose) of carboplatin intraperitoneally administered to mice is 150 mg/kg. Therefore, in the case of a mouse having a body weight of 30 g, the LD50 is 4.5 mg. The amount of carboplatin contained in 0.05g of the 30-fold amount of carboplatin-containing carrier was 7.5 mg, which means that carboplatin was implanted in a larger amount than LD50. However, the mice M169, F166, and F179 lived and were successfully treated for cancer. It is indicated that even when the amount of carboplatin exceeded LD50, the use of the carrier allowed sustained release of carboplatin and therefore reduced side effects.

### Summary

In the experiment using neuroblastoma model mice, mice to which carboplatin was directly administered died from serious side effects in several days before the end of life. On the other hand, mice implanted with the cotton-like carrier lived beyond a life-span of 8 weeks without side effects and were euthanized at 12 weeks of age for autopsy. As a result of pathological examination, no cancer cells were observed.

Therefore, the cotton-like carrier made it possible to locally administer an anticancer agent without causing systemic side effects and to kill cancer cells.

From the above, the anticancer agent-carrying bioabsorbable cotton-like material is very effective as a novel drug delivery (DDS) material.

### INDUSTRIAL APPLICABILITY

As described above, the biodegradable fibers according to the present invention can provide a drug formulation material that is capable of locally and sustainably releasing a drug at any site in the body for a long period of time, that has bioabsorbability, and that is absorbed and broken down by the body after sustained release of the drug.

Further, implantation of the drug formulation in a patient can produce a therapeutic/preventive effect to enhance QOL (Quality of Life) without causing systemic side effects.

## Claims

1. A bioabsorbable cotton-like material having a cotton- or nonwoven fabric-like structure, comprising a fibrous material that comprises a drug and a biodegradable resin and has an average outer diameter of 1 µm or more but 150 µm or less.

2. The bioabsorbable cotton-like material according to claim 1, wherein the fibrous material has an average molecular weight of 50,000 or more but less than 1,000,000.

3. The bioabsorbable cotton-like material according to claim 1, whose bulk density is 0.01 g/cm³ to 0.1 g/cm³.

4. The bioabsorbable cotton-like material according to any one of claims 1 to 3, wherein the biodegradable resin is PLGA or a copolymer thereof.

5. The bioabsorbable cotton-like material according to any one of claims 1 to 4, wherein the drug is an anticancer agent.

6. The bioabsorbable cotton-like material according to any one of claims 1 to 5, which has been subjected to sterilization treatment.

7. A method for manufacturing a bioabsorbable cotton-like material, comprising:
step 1) dissolving a biodegradable resin and a drug in a solvent to prepare a spinning solution; and
step 2) spinning fibers from the spinning solution by electrospinning.

8. The method for manufacturing a bioabsorbable cotton-like material according to claim 7, wherein in the step 2), fibers are spun by electrospinning by applying a voltage between a nozzle part provided on a spinning solution extrusion side and a plate placed in an ethanol bath provided on a collector side to deposit a bioabsorbable cotton-like material in the ethanol bath to form a bioabsorbable cotton-like material having a cotton-like three-dimensional structure.

9. The method for manufacturing a bioabsorbable cotton-like material according to claim 7 or 8, further comprising step 3) sterilization treatment.

10. The method for manufacturing a bioabsorbable cotton-like material according to any one of claims 7 to 9, wherein the biodegradable resin is PLGA or a copolymer thereof, and the solvent is chloroform or dichloromethane.

11. The manufacturing method according to any one of claims 7 to 10, wherein the drug is an anticancer agent.

12. A method of treating or preventing a disease in the patient, comprising:
1) implanting the bioabsorbable cotton-like material according to claim 6 in a body of a patient;
2) sustainably releasing the drug from the bioabsorbable cotton-like material; and
3) treating or preventing the disease in the patient by an effect of the sustainably released drug.

13. The method according to claim 12, wherein the bioabsorbable cotton-like material according to claim 6 is implanted by laparotomy.

14. The method according to claim 12, wherein the bioabsorbable cotton-like material according to claim 6 is implanted by a minimally invasive medical procedure using an injector.

15. The method according to claim 12, wherein the drug is an anticancer agent, and the disease is cancer.

16. The method according to claim 15, wherein the patient has been subjected to resection of cancer tissue or cancer cells.

17. The method according to claim 15 or 16, wherein the cancer is malignant bone tumor.

18. A kit for use in the method according to claim 12 or 13, comprising the bioabsorbable cotton-like material according to claim 6.

19. A kit for use in the method according to claim 14, comprising an injector and the bioabsorbable cotton-like material according to claim 6.

20. The kit according to claim 19, wherein the bioabsorbable cotton-like material according to claim 6 is contained in the injector.
